# EUROPEAN PATENT SPECIFICATION

(11) **EP 1 788 980 B1**
(45) Date of publication and mention of the grant of the patent: **12.01.2011**
(21) Application number: 05809787.4
(22) Date of filing: 18.08.2005
(51) Int. Cl.: A61K 31/655, A61K 31/727, A61L 27/34, A61L 27/54, A61L 31/10, A61L 31/16, A61L 29/08, A61L 29/16

(54) **MULTI-FUNCTIONAL BIOCOMPATIBLE COATINGS FOR INTRAVASCULAR DEVICES**
BIOKOMPATIBLE MULTIFUNKTIONSBESCHICHTUNGEN FÜR INTRAVASKULÄRE VORRICHTUNGEN
REVETEMENTS BIOCOMPATIBLES MULTIFONCTIONNELS POUR DISPOSITIFS INTRAVASCULAIRES

(30) Priority: 23.08.2004 US 924102
(43) Date of publication of application: 30.05.2007
(73) Proprietor: THE REGENTS OF THE UNIVERSITY OF MICHIGAN, Ann Arbor, Michigan 48109-1280 (US); Michigan Critical Care Consultants, Inc., Ann Arbor, MI 48103 (US)
(72) Inventor: ZHOU, Zhengrong, Ann Arbor, MI 48104 (US); MEYERHOFF, Mark, E., Ann Arbor, MI 48103 (US); REYNOLDS, Melissa, M., Ann Arbor, MI 38103 (US)
(74) Representative: Gowshall, Jonathan Vallance
(86) International application number: PCT/US2005/029490
(87) International publication number: WO 2006/023693

(56) References cited:
- WO-A-03/011250
- WO-A-03/015677
- US-A1- 2003 203 915
- US-B1- 6 335 029
- US-B1- 6 841 166
- MOWERY K A ET AL: "Preparation and characterization of hydrophobic polymeric films that are thromboresistant via nitric oxide release" BIOMATERIALS, ELSEVIER SCIENCE PUBLISHERS BV., BARKING, GB, vol. 21, no. 1, 1 January 2000 (2000-01-01), pages 9-21, XP002218425 ISSN: 0142-9612

## Description

### BACKGROUND

Embodiments of the present invention are directed to biocompatible coatings, and methods for forming and using the same.

Polymeric materials used to construct or coat a wide variety of blood-contacting devices such as catheters, vascular grafts, extracorporeal circuits, and chemical sensors may be made of materials that exhibit good hemocompatibility. However, a persistent problem with these devices is that they may, in some instances, elicit a thrombogenic response *in vivo* when in direct contact with blood. The blood coagulation cascade is initiated by protein adsorption on the surface of the material, followed by platelet adhesion and activation. A series of coagulation factors then convert soluble fibrinogen to insoluble fibrin that entraps activated platelets, thus resulting in thrombus formation.

Numerous approaches aimed at developing more blood compatible polymeric materials have been investigated. One strategy includes immobilizing heparin onto the polymer surfaces of devices that come in contact with blood. However, thrombus formation is not completely eliminated. This may be due, in part, to the fact that the amount of heparin on the polymer surfaces may not be adequate to effectively prevent coagulation, and/or the immobilized species may not bind fully with antithrombin III and thrombin, simultaneously, generally necessary for inhibiting fibrin formation. Further, heparin does not inhibit platelet activity and may actually cause platelet activation to a certain extent.

Another approach to reduce or eliminate thrombus formation includes doping polymers with prostacyclin (PGI₂) or immobilizing PGI₂ to the polymer surface. However, these doped polymers do not exhibit enhanced blood compatibility *in vivo,* due to either insufficient amount of PGI₂ release or loss of biological function after immobilization.

Other approaches have been explored that attempt to modify polymer surfaces to achieve enhanced blood compatibility. These include thrombomodulin impregnating, endothelial cell (EC) seeding, as well as protein adhesion and cell adhesion suppression. Although these different approaches have all met with variable levels of success, none have been able to yield a polymeric material that is nonthrombogenic.

Nitric oxide (NO) has also been shown to have several important physiological functions, including its unique vasodilating properties, cancer-fighting potency, anti-platelet activity, and anti-cell proliferation attributes. Although NO is a stable radical, it may be highly reactive with hemoglobin and oxygen, thus making delivery of NO to the target site challenging. Indeed, many advances have been achieved using water-soluble NO donors/adducts or NO generators as NO delivery agents. For example, the diazeniumdiolated proline (PROLI/NO), when infused into blood, has been shown to relieve muscle spasms. In addition, it has been reported that dimethylene triamine diazeniumdiolates (DETA/NO) substantially suppress overproliferation of cells after vascular injury, and glycosylated diazeniumdiolates possess anti-tumor activity.

However, the use of such water-soluble diazeniumdiolates and other NO donors with hydrophobic matrices to form biocompatible coatings may be problematic. For example, (Z)-1-[N-methyl-N-[6-(N-methylammoniohexyl)amino]]-diazen-1-ium-1,2-diolate (MAHMA/NO) dispersed in a silicone rubber matrix may, in some instances, prevent thrombus formation on the surface of intravascular sensors. The same compound may greatly reduce platelet activity when employed within a polymer coating on the inner walls of extracorporeal circuits. However, MAHMA/NO and its corresponding diamine precursor tend to leach from the surface of the polymer matrix and back react with an oxidative intermediate of NO to form potentially toxic nitrosamines.

### SUMMARY

Embodiments of the present invention substantially solve the drawbacks enumerated above by providing a polymeric coating that is adapted to substantially eliminate thrombus formation when in contact with blood. The polymeric coating includes a first polymeric layer and a second polymeric layer. Interposed between the first and second polymeric layers is a polymeric matrix layer doped with at least one of a nitric oxide donor and a nitric oxide generator. The nitric oxide donor and/or the nitric oxide generator are/is capable of releasing or generating NO. A bioactive agent is immobilized to the surface of the second polymeric layer.

### BRIEF DESCRIPTION OF THE DRAWINGS

Objects, features and advantages of embodiments of the present invention will become apparent by reference to the following detailed description and drawings, in which:
Fig. 1A is a semi-schematic view of an embodiment of the polymeric coating on a substrate;
Fig. 2A is a semi-schematic view of an alternate embodiment of the polymeric coating on a substrate;
Fig. 2B depicts alternate embodiments of the immobilized bioactive agents;
Fig. 3A is a semi-schematic top view of an embodiment of a polymeric coating on an inner surface of a substrate;
Fig. 3B is a semi-schematic top view of an alternate embodiment of a polymeric coating on an outer surface of a substrate;

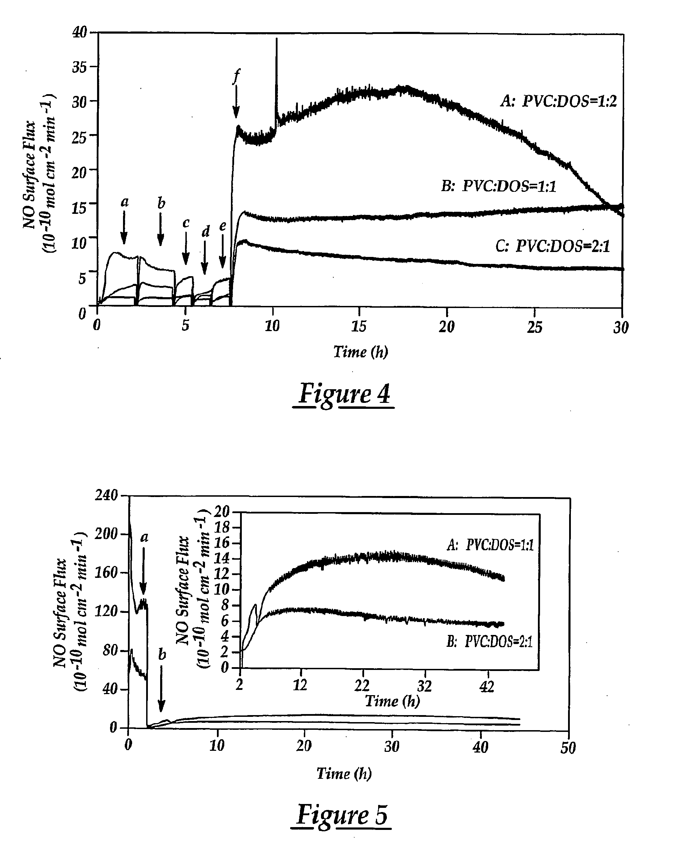

Fig. 4 is a chart depicting the NO surface flux generated from SR catheter sleeves (d=0.1cm, h=2.5 cm) coated with plasticized PVC incorporated with KTpClPB and 4% DBHD/NO and top coated with PVC-NH₂ during and after heparin immobilization (A - PVC:DOS=1:2 with top-coating, B - PVC:DOS=1:1 with top-coating, C - PVC:DOS=2:1 with top-coating; a - equilibrated with MES at 25°C, b - reacted with Hep/EDC/NHS in MES at 25°C, c - washed with Na₂HPO₄ at 25°C, d - washed with 4M NaCl at 25°C, e - washed with water at 25°C, f - soaked in PBS at 37°C; A(a+b+c+d+e)=~5% of total NO, B(a+b+c+d+e)=~2% of total NO, and C(a+b+c+d+e)=less than 1% of total NO); and
Fig. 5 is a chart depicting the NO surface flux generated from SR catheter sleeves (d=0.1cm, h=2.5cm) coated with plasticized PVC incorporated with KTpClPB and 4% DBHD/N₂O₂ and top coated with PVC-NH₂ during and after heparin immobilization; (A-PVC:DOS=1:1 with top-coating, B---PVC:DOS=2:1 with top-coating; a - immobilize Hep-CHO (pH=3.5) at 50°C, b - soaked in PBS at 37°C; A(a)= ~27% of total NO and B(a)= ~12% of total NO).

### DETAILED DESCRIPTION

Embodiment(s) of the polymeric coating may advantageously be used on intravascular devices, such as, for example, grafts, stents, catheters, extracorporeal circuits, and chemical sensors. As discussed above, prior attempts have been made to eliminate thrombus formation (ie. incorporation of NO donors) associated with intravascular devices, however, problems associated with these various techniques have yet to be overcome.

The effectiveness of NO release or NO generating coatings may be further enhanced by adding additional endothelial cell (EC) agents to the surface of these coatings. For example, the level of NO flux required for a given polymer film to exhibit reduced thrombosis may be lowered when a suitable synergistic agent is linked to the surface (e.g, thrombomodulin), or is released from the surface (e.g. prostacyclin). Without being bound to any theory, it is believed that engineering films with lower flux may advantageously enable a prolonged release of the NO, and thus the benefits of NO may be extended beyond what may be achieved using an NO release coating alone (a non-limitative example of which includes coatings with lipophilic diazeniumdiolates).

Embodiment(s) of the polymeric coatings may advantageously mimic non-thrombogenic EC which line the inner walls of all healthy blood vessels, and may advantageously use chemical surface moieties which suppress blood-material interactions (e.g., polymeric surfaces that exhibit decreased protein and cell adhesion). Without being bound to any theory, it is believed that mimicking the endothelial cells and having the various species working synergistically may prevent thrombosis and stenoses. Molecules contributing to the non-thrombogenic and anti-cell proliferation properties of the EC include nitric oxide (NO), prostacyclin (PGI₂), thrombomodulin (TM), and heparan sulfates. Incorporating these agents into or on polymer matrices such that they are either released from polymers or present in biologically active forms on the polymer surfaces, forms embodiments of the multi-functional coatings that are substantially truly biomimetic and that mimic the EC in functionality results. In one embodiment of the multi-functional polymeric coating, two or more of these naturally occurring EC derived anti-platelet, anticoagulation and/or anti-cell proliferation agents are included.

Therefore, embodiment(s) of the coatings described herein substantially solve lingering thrombosis and restenosis problems associated with placement of intravascular devices. As described herein, embodiment(s) of the polymeric coatings advantageously model the endothelial cells that line blood vessels in the human body, which cells have exceptional thromboresistivity. It is believed, without being bound to any theory, that the combination of NO release from nitric oxide donors (non-limitative examples of which include discrete and polymeric diazeniumdiolates) or generators (a non-limitative example of which includes a copper(II/I) ion-/copper metal-containing polymer matrix) and of naturally occurring endothelial cell agents in/on the polymeric coating substantially fully eliminates thrombus formation when the device is put in contact with blood. It is believed that this may be due, at least in part, to the substantially enhanced biocompatibility of the blood and the materials used in the coatings. Further, embodiment(s) of the coatings may also include combining NO-release or generation with drug delivery of other non-naturally occurring species that may assist in the prevention of restenosis, thrombosis, inflammation, and/or the like.

As used herein, the term "NO donors" generally refers to agents that have an NO-releasing moiety covalently bonded thereto such that the chemical functionality/functionalities release NO when exposed to appropriate conditions. The term "NO generators" generally refers to agents that directly cause NO to be produced from reagents, without an NO-releasing moiety covalently bonded thereto.

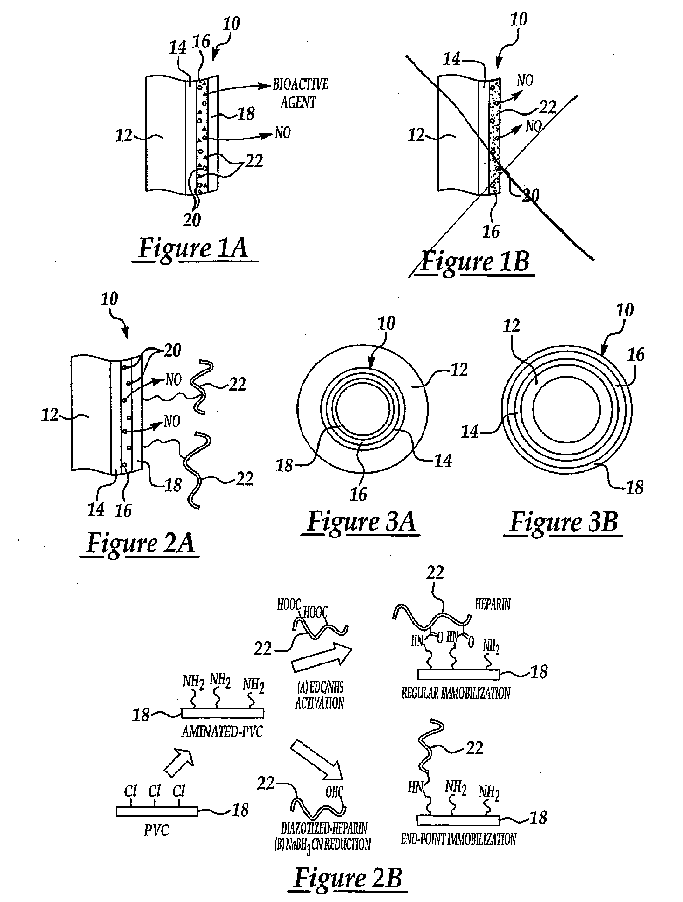

Referring now to Fig. 1A, an embodiment of an intravascular device 12 having a polymeric coating 10 thereon is depicted. It is to be understood that any suitable intravascular device 12 may be used, including, but not limited to grafts, stents, catheters, extracorporeal circuits, chemical sensors, and the like.

The polymeric coating 10 may include two or more layers attached to each other and in intimate contact therewith. Various hydrophobic polymeric materials may be employed in the coating 10 layer(s) 14, 16, 18 as disclosed herein. These include, but are not limited to materials such as poly(vinyl chloride) (PVC), silicone rubbers (SR), polyurethanes (PU), polymethacrylates, polyacrylates, polycaprolactones, polylactide, polyglycolide, poly(lactide-co-glycolide), poly(N-isopropyl acrylamide), polyacrylamides, copolymers thereof, and/or mixtures thereof. It is to be understood that each of the layers 14, 16, 18 may be polymeric matrices. It is to be further understood that the polymeric matrices may be plasticized or not, as desired. Further, the polymer of choice for the outer layers 16, 18 will generally be one capable of releasing NO from, for example, covalently attached and/or dispersed diazeniumdiolate type NO-adducts.

In an embodiment, a layer 14 (i.e. first layer) is established on the device 12. This layer 14 includes a polymeric material that is adapted to substantially prevent migration into device 12 of any compounds that may be present in subsequent layer(s) 16, 18 and/or to substantially promote adhesion between the layers 14, 16, 18 in the coating 10. Suitable non-limitative examples of the polymeric material used in layer 14 include poly(vinyl chloride), poly-p-xyxylenes, silicone rubbers, polyurethanes, polymethacrylates, poly(N-isopropyl acrylamide), polyacrylamides, primer compounds, and mixtures thereof. It is to be understood that the layer 14 may be a dense layer of the polymeric material. In an embodiment, the density of the layer 14 ranges between about 10 angstroms and about 100 micrometers.

Embodiment(s) of the polymeric coating 10 further include an active layer 16 (i.e. polymeric matrix layer) disposed on the layer 14. The active layer 16 may include a polymeric matrix material. It is to be understood that the polymeric matrix material may be plasticized. Non-limitative examples of polymeric matrix materials include plasticized poly(vinyl) alcohol, poly(vinyl chloride) (PVC), silicone rubbers (SR), polyurethanes (PU), polymethacrylates, polyacrylates, polycaprolactones, polylactide, polyglycolide, poly(lactide-co-glycolide), poly(N-isopropyl acrylamide), polyacrylamides, copolymers thereof, and/or mixtures thereof.

The active layer 16 includes NO donors/adducts or generators 20. It is to be understood that the addition of the NO donors or NO generators 20 to the active layer 16 may occur either simultaneously with or sequential to the establishment of the active layer 16 on the device 12.

In one non-limitative embodiment, the NO generator 20 is a copper(II/I) ion/copper metal or other transition metal ion- and/or metal-containing polymer matrix. Suitable non-limitative examples of other metals and/or metal ions which can form suitable metal ion-containing polymer matrices include calcium, magnesium, cobalt, manganese, iron, molybdenum, vanadium, aluminum, chromium, zinc, nickel, other transition metals, ions thereof, and/or mixtures thereof. Specific examples of the copper(II/I) ion/metal-containing polymer matrix include, but are not limited to copper(II)dibenzo[e,k]-2,3,8,9-tetraphenyl-1,4,7,10-tetraaza-cyclododeca-1,3,7,9-tetraene (copper(II)-DTTCT), copper(II)-cyclen and its polymeric derivatives, copper phosphate, metal copper, and the like, and mixtures thereof.

In another embodiment, the NO donors 20 are selected from discrete NO adducts and polymeric NO adducts. It is to be understood that the NO adduct 20 of choice is also one capable of spontaneous release of NO when the polymer is exposed to solutions and/or blood under physiological conditions. Some non-limitative examples of NO adducts 20 include protected and discrete *N-*diazeniumdiolates, polymer-based *N-*diazeniumdiolates, nitrosothiols, organic nitrates, metal-nitrosyls, C-based diazeniumdiolates, and/or mixtures thereof.

Spontaneous release of NO from the polymer may be governed by at least one process occurring between the NO adduct 20 and the aqueous environment. These include, but are not limited to at least one of diffusion and ionization of water or other blood components into/within the organic polymer; ion-exchange between the buffer ions and ions within the polymer; protonation of amine-nitrogen-bearing compounds to yield NO; and deprotonation of water by secondary amine sites to yield organic ammonium hydroxides or by sodium or other metal ions to yield metal hydroxides.

"Discrete NO adducts" as referred to herein are those compounds that have the NO-releasing moiety covalently attached to a small molecule or to a polymer filler (e.g., functionalized silica particles or titanium particles). It is to be understood that discrete NO adducts are generally not polymers. Those compounds that have their NO-releasing moiety covalently attached to a polymer backbone are generally referred to as "polymeric NO adducts." Non-limitative examples of suitable polymeric NO adducts include, but are not limited to, diazeniumdiolated silicone rubbers (DACA/N₂O₂), diazeniumdiolated methacrylates, diazeniumdiolated polyurethanes, diazeniumdiolated poly(vinyl chloride), and/or mixtures thereof. It is to be understood that generally neither the discrete NO adducts nor the polymeric NO adducts has a protecting group(s) attached thereto. However, in an embodiment in which the discrete NO adducts and/or polymeric NO adducts have a benign protecting group, it is to be understood that when the protecting group is released, a benign species is yielded. Still further, the benign protecting group of an NO adduct or a polymeric adduct may be removed prior to and/or during NO release. Furthermore, if a protecting group is utilized that is non-benign, it is to be understood that the protecting group is removed prior to application of the device (e.g. NO release).

It is to be further understood that discrete nitric oxide adducts may be either covalently attached to the polymer matrix or may be dispersed therein. Some examples of discrete diazeniumdiolates include, but are not limited to anionic diazeniumdiolates stabilized with metal cations, zwitterionic diazeniumdiolates, and protected discrete diazeniumdiolates (e.g. O² protected discrete diazeniumdiolates). In an embodiment incorporating protected nitric oxide adducts 20 (such as protected *N-*diazeniumdiolates) or nitric oxide generators 20, it is to be understood that the protected nitric oxide adducts 20 may be dispersed substantially throughout the polymer matrix.

Embodiments of the present method substantially avoid the toxicity of the NO release precursor materials while maintaining controlled NO fluxes. This may be accomplished by increasing the lipophilicity of the discrete diazeniumdiolate molecules, and/or covalently attaching such moieties directly to polymers (e.g., silicone rubbers, methacrylate-based polymers, etc.). The thomboresistivity of such polymers may be improved, with a significant decrease in platelet adhesion and activation on the surface of such materials when tested *in vivo.*

Non-limitative examples of NO donors 20 used to prepare an embodiment of the polymeric coating are diazeniumdiolates derived from dialkyl hexamethylene diamine compounds having the general linear structure: 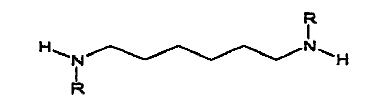 to form corresponding N-diazeniumdiolate derivatives having the general formula: 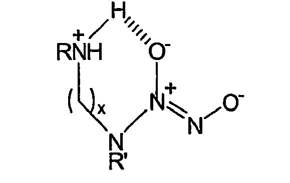 in which R is an alkyl group having one to twelve carbon atoms or a branched side chain. It is to be understood that the R groups may be different in character. For example, one R group may be a propyl group while another R group may be a butyl group. In an embodiment, the R groups may be hydrogen. Still further, the methylene spacer present between the amines in the derivatives may range from x=1 to x=6.

Other non-limitative examples of parent structures used to form diazeniumdiolates may be any primary or secondary amine containing compounds, including, but not limited to: 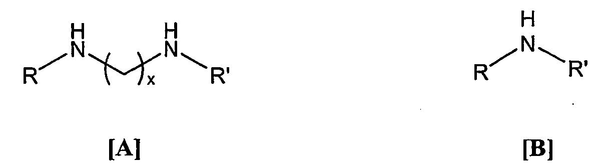 where R and R' may be hydrogen; n-alkyls; branched alkyls; aliphatics; cyclic and/or aromatic amine side-chains; ketones; aldehydes; amides; ether; esters; alkenes; alkynes; and/or mixtures thereof; and/or the like. Examples of the diazeniumdiolates that may be formed from parent structure A include the following:

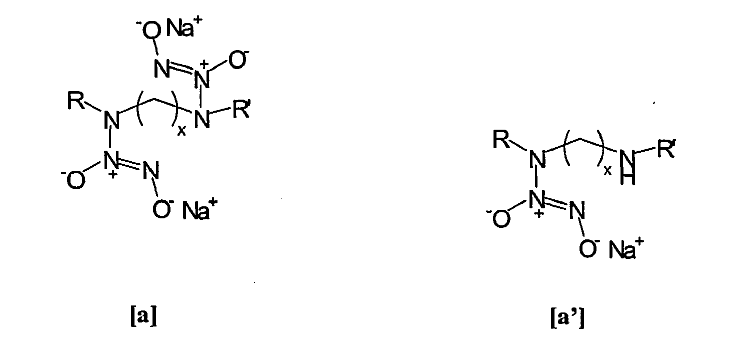

Examples of the diazeniumdiolates that may be formed from parent structure B include the following:

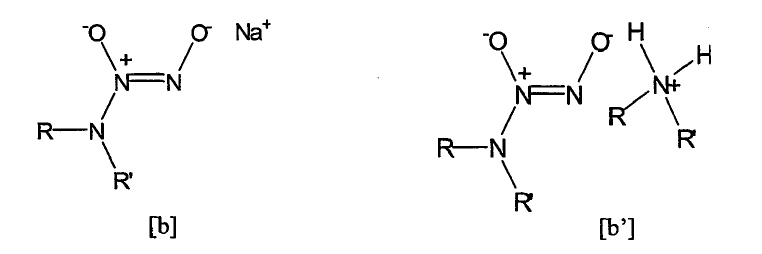

As a non-limitative example, a sodium ion is depicted in structures a, a', b, and b' as a counter ion in order to stabilize the respective diazeniumdiolates. It is to be understood that other metal ions such as ions of lithium, potassium, copper, and/or the like, and/or mixtures thereof, may be valid metal cations to stabilize the species.

As depicted, diazeniumdiolates with the previously mentioned diamine backbone or compounds containing one amine site or those containing three or more amine sites may be used in an embodiment of the active layer 16 of the polymeric coating 10. In an embodiment, one mole of the diazeniumdiolate species readily dissociates into two moles of NO_{(g)} and one mole of the donor amine when exposed to water or at relatively high temperature.

In one embodiment (as depicted in Figs. 1A, the active layer 16 may have, in addition to the NO donor or generator 20, one or more bioactive agents 22 incorporated therein. The bioactive agent 22 may be an anti-coagulant agent, an anti-platelet agent, anti-cell proliferators, anti-microbial, anti-viral, and/or mixtures thereof. Specific bioactive agents 22 include, but are not limited to heparin, heparan, heparan sulfate, prostacyclin, thrombomodulin, and/or mixtures thereof. In this embodiment, it is to be understood that the bioactive agent 22 may be substantially evenly dispersed throughout the active layer 16. In an embodiment, the bioactive agent(s) 22 is dispersed within and released from within the active layer 16.

The polymeric coating 10 includes a top layer 18 (e.g. second polymeric layer). The top layer 18 may act as a barrier layer to assist in controlling the release of the nitric oxide and/or other bioactive agents 22 located in the active layer 16. In addition, the top layer 18 may also prevent the active layer 16 from being directly exposed to the blood. Without the top layer 18, the agents (NO donors or generators 20 and/or bioactive agents 22) that are doped into the polymer matrix may, in some instances, interact with the blood (i.e., form a charge on the surface of the polymer or aggregate at the surface, etc.). It is to be understood, however, that in embodiments having the bioactive agents 22 dispersed within the active layer 16, a top layer 18 is used (Fig. 1A) as the release of the NO and/or bioactive agent 22 may be controlled by the polymer matrix into which they are incorporated.

The polymeric coating 10 includes the bioactive agent 22 dispersed (not shown) throughout the top polymeric layer 18. It is to be understood that this embodiment may include the NO donors 20 (a non-limitative example of which includes discrete *N-*diazeniumdiolates) or the NO generators 20 dispersed throughout the active layer 16.

Referring now to Fig. 2A, an alternate embodiment of the polymeric coating 10 is disclosed. As depicted, the polymeric coating 10 includes the top layer 18. A bioactive agent 22 is immobilized at the surface of the top layer 18. Suitable bioactive agents 22 that may be immobilized to the surface of the polymeric coating 10 via the top layer 18 include, but are not limited to heparin, heparan, prostacyclin, thrombomodulin, and mixtures thereof.

Various methods may be employed to immobilize the bioactive agent 22 to the surface of the polymeric coating 10. Fig. 2B depicts non-limitative examples of two alternate methods of achieving immobilization of the bioactive agents 22 (e.g. heparin). Each of the alternate methods includes first forming surface functional groups (e.g. NH₂ groups for binding heparin or COOH groups for binding thrombomodulin) on the top layer 18 that are adapted to covalently and/or ionically bond to the selected bioactive agent(s) 22. In an embodiment, the bioactive agent 22 may be bonded to the surface via a linker (a non-limitative example of which is hexamethylene diisocyanate (HMDI) which may link thrombomodulin to an aminated surface). In the examples depicted in Fig. 2B, the bioactive agent 22 is heparin, and the top layer 18 is aminated to form NH₂ groups. Generally, regular immobilization is formed by immobilizing the bioactive agent 22 via amide linkages; and end-point immobilization is formed by immobilizing the bioactive agent 22 via amine linkages. In the first non-limitative example depicted in Fig. 2B, EDC/NHS activated heparin is immobilized onto the aminated PVC surface via amide linkages to form immobilization; and in the second non-limitative example depicted in Fig. 2B, the terminal aldehyde groups of the diazotized heparin are reacted to the surface bearing primary amines, followed by reduction using sodium cyanoborohydride to form end-point immobilized heparin via amine linkages. It is to be understood that the bioactive agent 22 may be randomly or uniformly immobilized on the surface of the top layer 18. However, in either of the examples of immobilization described hereinabove, the bioactive agent 22 generally does not form a continuous or uniform layer on the top layer 18.

Fig. 3A is a top view of an embodiment of the polymeric coating 10 established on an inner surface of device 12. As depicted, the polymeric coating 10 includes the layer 14 directly established on the inner surface of the device 12, the polymeric matrix layer 16 established next, and the top layer 18 established such that it provides an outer surface to the coating 10.

Fig. 3B is a top view of an alternate embodiment of the polymeric coating 10 established on an outer surface of device 12. As depicted, the polymeric coating 10 includes the layer 14 directly established on the outer surface of device 12, the polymeric matrix layer 16 established next, and the top layer 18 established such that it provides an outer surface to the coating 10.

It is to be understood that the embodiments of the polymeric coatings 10 described herein are not limited to NO donors/generators in combination with one other anti-coagulant or anti-platelet agent. The NO donors/generators may be used in combination with two or more other anti-coagulants and/or anti-platelet agents (non-limitative examples of which include NO+Heparin+Prostacyclin; NO+Heparan+Thrombomodulin; NO+ Prostacyclin+ Thrombomodulin, NO+ Heparin+Prostacyclin+Thrombomodulin, etc.).

Further, each of the layers 14, 16, 18 may be established by any suitable technique that uses a polymer/solvent mixture for deposition. Several non-limitative examples of such techniques include spin coating, dip coating, spray coating, curtain coating, electrocoating, and the like. Other techniques that may be used include chemical vapor deposition (CVD) and plasma polymerization. However, it is to be understood that CVD and plasma polymerization techniques are generally not desirable to deposit the active layer 16. Further, plasma polymerization is generally not desirable to deposit the top layer 18 due to the possibility of reactive side products forming as a result of the deposition.

### Experimental

**Heparin immobilized polymeric films doped with NO donors. Heparin is a widely used** inhibitor, due in part to the fact that its binary complex with antithrombin III binds and inhibits both factor Xa and thrombin, two proteases that are desirable for the ultimate conversion of fibrinogen to fibrin.

The inner wall of the PVC tubing (or outer surface of SR catheter sleeves) were coated with a PVC layer (44 mg/mL in THF), followed by a plasticized PVC layer (133 mg poly(vinyl chloride), 66 mg dioctyl sebacate (DOS) and 15.7 mg KTpClPB in 3-4 mL of THF) doped with 10 mg of NO donor (N,N-dibuylhexamethylene diamine diazeniumdiolate or polymer-based NO donors), and finally with an aminated-PVC top coating (44 mg/mL in THF). The NO donor amount as well as the PVC/DOS ratios may be varied according to the desired applications. The aminated-PVC may be synthesized by (A) reacting PVC with various diamines such as 1,6-diaminohexane, 1,12-diaminododecane or polyethylene oxide (PEO) capped with amino groups or by (B) reacting carboxylated PVC, pre-activated with EDC/NHS (1-ethyl-3-(3-dimethylaminopropyl)carbodiimide hydrochloride) and N-hydroxysuccinimide), with the various diamines shown above. Heparin was covalently immobilized onto the device via (A) EDC/NHS activated immobilization or (B) end-point immobilization using NaBH₃CN reduction (also see Fig. 2B).
(A) The PVC tubing (or SR catheter sleeve) coated with aminated-PVC was equilibrated with MES-buffer (0.05M, pH 5.6) for about 30 min. Carboxylic acid groups of heparin (Hep-COOH) were activated using EDC (carbodiimide) and NHS (N-hydroxysuccinimide) in MES-buffer. After pre-activation for about 10 min, the coated PVC tubing was added to the EDC/NHS activated heparin solution. After about 2 hours of reaction, the inner wall of the heparinized tubing (or the outer surface of the SR catheter sleeve) was washed with 0.1 M Na₂HPO₄ 4 M NaCl and distilled water. The heparinized surface was quickly dried by flushing nitrogen onto it.
(B) The PVC tubing (or SR catheter sleeve) coated with aminated-PVC was reacted with diazotized-heparin (10 mg/mL) at 50°C for about 2 hours in the presence of NaBH₃CN (1 mg/mL). The solution pH was adjusted to 3.5 using NaOH/HCl. After the reaction, the inner wall of the heparinized tubing (or the outer surface of the SR catheter sleeve) was washed with 0.1 M Na₂HPO₄, 4 M NaCl and distilled water. The heparinized surface was quickly dried by flushing nitrogen onto it.

The NO release was measured during and after each immobilization. It was found that the surface NO-release of the coating was not interfered with by the surface bound heparin, and the surface NO flux can be maintained at above 10×10⁻¹⁰ mol·cm⁻²·min⁻¹ for at least 24 hours (Figs. 4 and 5). Fig. 5 also depicts an insert blow-up graph showing the NO flux after 2 hours of heparin immobilization. The surface bound heparin was evaluated with the antifactor Xa assay and showed anti-coagulant activity (0.1-100 mIU/cm⁻² polymer surface).

## Claims

1. A polymeric coating for an intravascular device, comprising:
a first polymeric layer;
a second polymeric layer comprising a bioactive agent, wherein the bioactive agent is immobilized on a surface of the second polymeric layer;
a polymeric matrix layer interposed between the first and second polymeric layers, the polymeric matrix layer comprising a nitric oxide donor or a nitric oxide generator;
wherein the polymeric coating is adapted to substantially eliminate thrombus formation when in contact with blood.

2. The polymeric coating as defined in claim 1 wherein immobilization is accomplished by at least one of covalent bonding and ionic bonding.

3. The polymeric coating as defined in claim 1 or 2 wherein the polymeric matrix layer is disposed on the first polymeric layer;
the second polymeric layer is disposed on the polymeric matrix layer.

4. The polymeric coating as defined in claim 3 wherein the second polymeric layer is aminated.

5. The polymeric coating as defined in any one of claims 1 to 4 wherein the bioactive agent is at least one of an anticoagulant agent, an anti-platelet agent, an antiproliferative agent, an antimicrobial agent, and mixtures thereof.

6. The polymeric coating as defined in any one of claims 1 to 5 wherein the bioactive agent is selected from heparin, heparan, prostacyclin, thrombomodulin, and mixtures thereof.

7. The polymeric coating as defined in any one of claims 1 to 6 wherein at least one of the first and second polymeric layers is independently selected from poly(vinyl chloride), silicone rubbers, polyurethanes, polymethacrylates, polyacrylates, polycaprolactones, polylactide, polyglycolide, poly(lactide-co-glycolide), poly(N-isopropyl acrylamide), polyacrylamide, copolymers thereof, and mixtures thereof.

8. The polymeric coating as defined in any one of claims 1 to 7 wherein the nitric oxide generator is a metal-/metal ion-containing polymer matrix.

9. The polymeric coating as defined in any one of claims 1 to 8 wherein the nitric oxide generator comprises at least one of copper, calcium, magnesium, cobalt, manganese, iron, molybdenum, vanadium, aluminum, chromium, zinc, nickel, ions thereof, and mixtures thereof, preferably
wherein the metal-/metal ion-containing polymer matrix comprises copper(II)dibenzo[e,k]-2,3,8,9-tetraphenyl-1,4,7,10-tetraaza-cyclododeca-1,3,7,9-tetraene, copper(II)-cyclen and polymeric derivatives thereof, copper phosphate, metal copper, and mixtures thereof, or
wherein the nitric oxide donor is at least one of a discrete nitric oxide adduct and polymeric nitric oxide adduct.

10. The polymeric coating as defined in claim 9 wherein the discrete nitric oxide adduct is selected from *N-*diazeniumdiolates, nitrosothiols, organic nitrates, metal-nitrosyls, C-based diazeniumdiolates, and mixtures thereof, preferably discrete *N-*diazeniumdiolates.

11. The polymeric coating as defined in claim 10 wherein the discrete *N-*diazeniumdiolates is selected from anionic diazeniumdiolates stabilized by metal cations, zwitterionic diazeniumdiolates, and mixtures thereof.

12. The polymeric coating as defined in claim 9 wherein the polymeric nitric oxide adduct comprises polymer-based *N-*diazeniumdiolates.

13. A device comprising a polymeric coating as defined in any preceding claim.

14. A device as defined in claim 13, wherein the device is an intravascular device.

15. A device according to claim 14 or 15 for use in therapy.

16. An intravascular device according to Claim 15 for use in a method for substantially eliminating thrombus formation, the method comprising inserting said intravascular device into a patient, wherein the polymeric coating is adapted to substantially eliminate at least one of thrombus formation and restenosis when in contact with blood.

## Patentansprüche

1. Polymerbeschichtung für eine intravaskuläre Vorrichtung, die umfasst:
eine erste Polymerschicht;
eine zweite Polymerschicht, die ein bioaktives Mittel umfasst, wobei das bioaktive Mittel auf einer Oberfläche der zweiten Polymerschicht immobilisiert wird;
eine zwischen der ersten und der zweiten Polymerschicht angeordnete Polymermatrixschicht, wobei die Polymermatrixschicht einen Stickstoffmonoxiddonor oder einen Stickstoffmonoxidgenerator umfasst;
wobei die Polymerbeschichtung ausgebildet ist, um bei Kontakt mit Blut eine Thrombusbildung im Wesentlichen zu eliminieren.

2. Polymerbeschichtung gemäß Anspruch 1, wobei die Immobilisierung durch eine kovalente Bindung und/oder eine Ionenbindung erreicht wird.

3. Polymerbeschichtung gemäß Anspruch 1 oder 2, wobei die Polymermatrixschicht auf der ersten Polymerschicht angeordnet ist;
die zweite Polymerschicht auf der Polymermatrixschicht angeordnet ist.

4. Polymerbeschichtung gemäß Anspruch 3, wobei die zweite Polymerschicht aminiert ist.

5. Polymerbeschichtung gemäß einem der Ansprüche 1 bis 4, wobei das bioaktive Mittel ein Antikoagulans und/oder ein Antithrombozytenmittel und/oder ein antiproliferatives Mittel und/oder ein antimikrobielles Mittel und/oder Mischungen davon ist.

6. Polymerbeschichtung gemäß einem der Ansprüche 1 bis 5, wobei das bioaktive Mittel aus Heparin, Heparan, Prostacyclin, Thrombomodulin und Mischungen davon ausgewählt ist.

7. Polymerbeschichtung gemäß einem der Ansprüche 1 bis 6, wobei die erste und/oder die zweite Polymerschicht unabhängig ausgewählt ist aus Poly(vinylchlorid), Silikongummis, Polyurethanen, Polymethacrylaten, Polyacrylaten, Polycaprolactonen, Polylactid, Polyglycolid, Poly(lactid-co-glycolid), Poly(N-isopropylacrylamid), Polyacrylamid, Copolymeren davon und Mischungen davon.

8. Polymerbeschichtung gemäß einem der Ansprüche 1 bis 7, wobei der Stickstoffmonoxidgenerator eine metall-/metallionenhaltige Polymermatrix ist.

9. Polymerbeschichtung gemäß einem der Ansprüche 1 bis 8, wobei der Stickstoffmonoxidgenerator Kupfer und/oder Kalzium und/oder Magnesium und/oder Cobalt und/oder Mangan und/oder Eisen und/oder Molybdän und/oder Vanadium und/oder Aluminium und/oder Chrom und/oder Zink und/oder Nickel und/oder Ionen davon und/oder Mischungen davon umfasst, bevorzugt
wobei die metall-/metallionenhaltige Polymermatrix Kupfer(II)dibenzo[e,k]-2,3,8,9-tetraphenyl-1,4,7,10-tetraaza-cyclododeca-1,3,7,9-tetraen, Kupfer(II)-Cyclen und Polymerderivate davon, Kupferphosphat, Metallkupfer und Mischungen davon umfasst oder
wobei der Stickstoffmonoxiddonor ein diskretes Stickstoffmonoxidaddukt und/oder ein polymeres Stickstoffmonoxidaddukt ist.

10. Polymerbeschichtung gemäß Anspruch 9, wobei das diskrete Stickstoffmonoxidaddukt aus *N-*Diazeniumdiolaten, Nitrosothiolen, organischen Nitraten, Metallnitrosylen, C-basierten Diazeniumdiolaten und Mischungen davon, bevorzugt diskreten *N-*Diazeniumdiolaten, ausgewählt ist.

11. Polymerbeschichtung gemäß Anspruch 10, wobei die diskreten *N-*Diazeniumdiolate aus anionischen Diazeniumdiolaten, die von Metallkationen stabilisiert werden, zwitterionischen Diazeniumdiolaten und Mischungen davon ausgewählt sind.

12. Polymerbeschichtung gemäß Anspruch 9, wobei das polymere Stickstoffmonoxidaddukt polymerbasierte *N-*Diazeniumdiolate umfasst.

13. Vorrichtung, die eine Polymerbeschichtung gemäß einem vorangehenden Anspruch umfasst.

14. Vorrichtung gemäß Anspruch 13, wobei die Vorrichtung eine intravaskuläre Vorrichtung ist.

15. Vorrichtung gemäß Anspruch 14 oder 15 zur Verwendung bei Therapien.

16. Intravaskuläre Vorrichtung gemäß Anspruch 15 zur Verwendung in einem Verfahren, um eine Thrombusbildung im Wesentlichen zu eliminieren, wobei das Verfahren umfasst, dass die intravaskuläre Vorrichtung in einen Patienten eingeführt wird, wobei die Polymerbeschichtung ausgebildet ist, um bei Kontakt mit Blut eine Thrombusbildung und/oder eine Restenose im Wesentlichen zu eliminieren.

## Revendications

1. Un enduit polymère pour un dispositif intravasculaire, comprenant :
une première couche polymère ;
une seconde couche polymère comprenant un agent bioactif, dans lequel l'agent bioactif est immobilisé à la surface de la deuxième couche polymère ;
une couche matrice polymère intercalée entre la première et la seconde couche polymère, la couche matrice polymère comprenant un donneur d'oxyde nitrique ou un générateur d'oxyde nitrique;
dans lequel l'enduit polymère est adapté pour éliminer de façon substantielle la formation de thrombus lorsqu'il est au contact du sang.

2. L'enduit polymère conforme à la définition de la revendication 1 dans lequel l'immobilisation est accomplie par au moins une liaison covalente ou une liaison ionique.

3. L'enduit polymère conforme à la définition de la revendication 1 ou 2 dans lequel la couche matrice polymère est déposée sur la première couche polymère ;
la seconde couche polymère est déposée sur la couche matrice polymère.

4. L'enduit polymère conforme à la définition de la revendication 3 dans lequel la seconde couche polymère est aminée.

5. L'enduit polymère conforme à la définition de l'une quelconque des revendications 1 à 4 dans lequel l'agent bioactif est au moins un des agents suivants : un agent anticoagulant, un agent anti-plaquettes, un agent antiproliférant, un agent antimicrobien, et les mélanges de ceux-ci.

6. L'enduit polymère conforme à la définition de l'une quelconque des revendications 1 à 5 dans lequel l'agent bioactif est choisi entre l'héparine, l'héparane, la prostacycline, la thrombomoduline, et les mélanges de celles-ci.

7. L'enduit polymère conforme à la définition de l'une quelconque des revendications 1 à 6 dans lequel au moins une des première et seconde couche polymère est choisie indépendamment parmi les polychlorures de vinyle, les caoutchoucs de silicone, les polyuréthanes, les polyméthacrylates, les polyacrylates, les polycaprolactones, le polylactide, le polyglycolide, le poly(lactide-co-glycolide), le poly(N-isopropyl acrylamide), le polyacrylamide, les copolymères de ceux-ci, et les mélanges de ceux-ci.

8. L'enduit polymère conforme à la définition de l'une quelconque des revendications 1 à 7 dans lequel le générateur d'oxyde nitrique est une matrice polymère contenant un métal/ion métallique.

9. L'enduit polymère conforme à la définition de l'une quelconque des revendications 1 à 8 dans lequel le générateur d'oxyde nitrique comprend au moins un des composés suivants : cuivre, calcium, magnésium, cobalt, manganèse, fer, molybdène, vanadium, aluminium, chrome, zinc, nickel, les ions et les mélanges de ceux-ci, préférablement
dans lequel la matrice polymère contenant un métal/ion métallique comprend du cuivre(II)dibenzo[e,k]-2,3,8,9-tetraphényl-1,4,7,10-tetraaza-cyclododéca-1,3,7,9-tetraène, du cuivre(II)-cyclène et les dérivés polymères de ceux-ci, le phosphate de cuivre, le cuivre métallique, et les mélanges de ceux-ci, ou
dans lequel le donneur d'oxyde nitrique est au moins un adduct d'oxyde nitrique distinct ou un adduct polymère d'oxyde nitrique.

10. L'enduit polymère conforme à la définition de la revendication 9 dans lequel l'adduct d'oxyde nitrique distinct est choisi parmi les *N-*diazeniumdiolates, les nitrosothiols, les nitrates organiques, les métaux nitrosylés, les diazeniumdiolates à base carbone, et les mélanges de ceux-ci, préférablement des *N-*diazeniumdiolates distincts.

11. L'enduit polymère conforme à la définition de la revendication 10 dans lequel les N-diazeniumdiolates distincts sont choisis parmi les diazeniumdiolates anioniques stabilisés par des cations métalliques, les diazeniumdiolates zwitterioniques, et les mélanges de ceux-ci.

12. L'enduit polymère conforme à la définition de la revendication 9 dans lequel l'adduct polymère d'oxyde nitrique comprend des *N-*diazeniumdiolates à base polymère.

13. Un dispositif comprenant un enduit polymère conforme à la définition de l'une quelconque des revendications précédentes.

14. Un dispositif conforme à la définition de la revendication 13, dans lequel le dispositif est un dispositif intravasculaire.

15. Un dispositif conforme à la définition de la revendication 14 ou 15 pour l'utilisation dans une thérapie.

16. Un dispositif intravasculaire conforme à la définition de la revendication 15 pour l'utilisation dans une méthode d'élimination substantielle de la formation de thrombus, la méthode comprenant insertion dudit dispositif intravasculaire chez un patient, dans lequel l'enduit polymère est adapté pour éliminer substantiellement au moins la formation de thrombus ou la resténose lorsqu'il est en contact avec le sang.
